# EUROPEAN PATENT SPECIFICATION

(11) **EP 2 898 853 B1**
(45) Date of publication and mention of the grant of the patent: **06.03.2019**
(21) Application number: 13839154.5
(22) Date of filing: 18.09.2013
(51) Int. Cl.: A61C 8/00, A61C 13/12, A61B 17/88

(54) **MECHANICAL TOOL**
MECHANISCHES WERKZEUG
OUTIL MÉCANIQUE

(30) Priority: 19.09.2012 ES 201230974
(43) Date of publication of application: 29.07.2015
(73) Proprietor: Servocad Microtronics S.L., 08272 Sant Fruitós de Bages (ES)
(72) Inventor: HERNANDEZ JUANPERA, Jesus, E-08241 Barcelona (ES)
(74) Representative: Isern Patentes y Marcas S.L.
(86) International application number: PCT/ES2013/070648
(87) International publication number: WO 2014/044889

(56) References cited:
- WO-A2-96/27345
- DE-A1-102010 004 031
- DE-A1-102010 004 031
- ES-T3- 2 322 900
- US-A- 5 105 690
- US-A- 5 690 489
- US-A- 5 885 299
- US-A1- 2004 225 292
- US-B1- 6 454 567

## Description

### PURPOSE OF THE INVENTION

The purpose of the present invention application is the registration of a mechanical tool that incorporates notable innovations and advantages.

More specifically, the invention proposes to develop a mechanical tool, particularly for the manipulation of small-sized objects such as dental implants, post-traumatic prostheses, screw, bolts, etc., that require precision and safety during their use.

### BACKGROUND OF THE INVENTION

A wide variety of mechanical tools for different applications and fields of the art are well known in the market.

In the particular case of the fields of dentistry and traumatology, and more specifically in the implantation of dental implants, screws, bolts etc., the use of instruments or tools that make it possible to grasp the implant in such a way that it can be inserted in place, for example, through the use of a magnet or with the aid of an end of the instrument having a specific contour is known. However, in practice it has been proven that this type of tool does not assure 100% fastening of the dental implant in the desired way since, due to a sudden movement, the implant can come detached from the instrument.

Also known are tools for the manipulation of objects that function pneumatically, such as, for example, patent No. ES 2114 148, which describes a pneumatic tool. However this tool has a complex structure with a large number of components, including the presence of a pneumatic motor, for which reason it is more costly to manufacture and provide with a source of compressed air, with the additional cost and maintenance of the latter.

DE 10 2010 004032 discloses a dental tool for the manipulation of a prosthetic element comprising a fist shaft and a second shaft which are oppositely orientated. Each shaft is provided with a geometric termination which can be coupled in a region of the object to be handled.

Hence, there is still the need to provide a manually or electrically activated mechanical tool that solves the problems discussed above.

### DESCRIPTION OF THE INVENTION

The present invention has been developed to provide a mechanical tool that is an innovation within its area of application and resolves the above-mentioned disadvantages, while bringing other additional advantages that will be evident from the description below.

Hence, it is the purpose of the present invention to provide a mechanical tool, particularly for the manipulation of small-sized objects, which is characterised essentially by the fact that it consists of a first elongated shaft that is interiorly hollow provided on its free end with a defined geometric termination that can be coupled in the region of the object to be handled and a second elongated shaft that passes through the inside of the first elongated shaft that has at its free end a defined geometric termination that projects outwardly from the first elongated shaft and can be coupled in a second region of the object to be handled, one of the two elongated shafts being able to rotate on its own central longitudinal axis with respect to the other elongated shaft, and including actuating means for carrying out such rotary action.

Thanks to these characteristics, a tool that is easy to build and simple to use can be used to handle small-sized objects that require precise manipulation, such as, for example, placing dental implants inside a patient's mouth, since the arrangement of two coupling points ensures that the object will not separate unintentionally, since one of the movable coupling points is a blocking element with respect to the other.

In a particularly preferred embodiment, the activation means have a push button that can be moved axially and are attached to the rotary shaft by means of a cam system. Specifically, the cam has a groove with a curved path that is located on a shaft that projects from the push button, and attached to that slot is a converter element joined to the elongated shaft as a unit and able to turn.

Advantageously, the tool includes an elastic means attached to the cam system, such as an substantially laminar body that is joined to the convertor element and the elongated rotary shaft.

Preferably, the protective casing has a region in the form of a handle that facilitates its manual manipulation.

In accordance with one embodiment, the geometric termination of the first elongated shaft has a polygonal exterior and/or interior contour.

Additionally, the geometric termination of the second shaft has a polygonal exterior contour that is complementary with the first shaft, so as to permit keeping their corresponding surfaces in parallel.

In a preferred embodiment, the geometric terminations of the first and second shafts have the same polygonal exterior and/or interior contour.

Preferably, the activation means are housed inside a protective casing from which the first and second elongated shafts project.

In an alternative embodiment of the invention, the protective casing may include a coupling region suitable for coupling an electric tool in such a way that the rotation of the rotary shaft is carried out with the aid of electrical means.

Other characteristics and advantages of the mechanical tool that is the subject matter of the present invention will be evident from the description of a preferred but not exclusive embodiment, which is illustrated by way of a non-limiting example in the drawings attached, in which:

### BRIEF DESCRIPTION OF THE DRAWINGS

Figure 1 is a perspective view of a first embodiment of the manually-operated mechanical tool in accordance with the present invention.
Figure 2 is a perspective view of the mechanical tool shown in figure 1 in which the two ends of each of the two elongated shafts are fitted into each other.
Figure 3 is an enhanced perspective view of the tool shown in figures 1 and 2 in which the protective casing is not shown for reasons of clarity.
Figure 4 is a perspective view of a second embodiment of the elastic means.
Figure 5 is a perspective view of a second embodiment of a mechanical tool in accordance with the invention, and
Figure 6 is a perspective view of an alternative embodiment of the tool designed to couple with an electrically-operated device.

### DESCRIPTION OF A PREFERRED EMBODIMENT

In light of the above-mentioned figures and in accordance with the adopted numbering, an example of a preferred embodiment of the invention can be seen, comprising the parts and elements indicated and described below in detail for use in the placement of dental implants, although such use should not be considered a limitation of its employment.

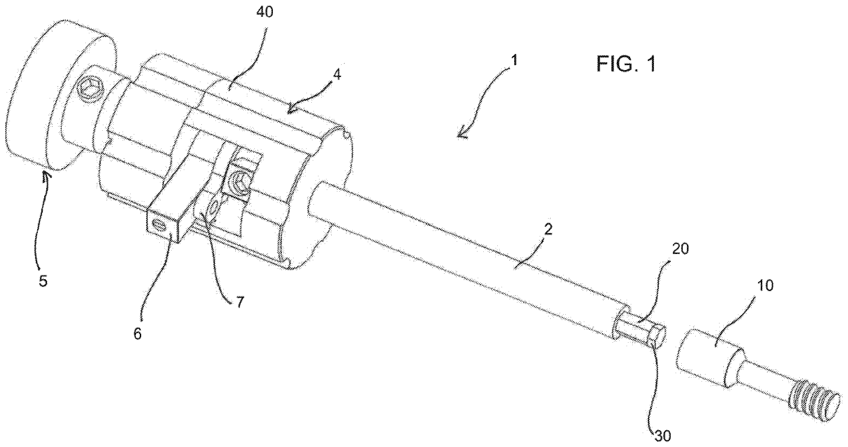

Thus, as seen in figure 1, a mechanical tool for the manipulation of smaller-sized objects, indicated in general as reference (1), consists of a first elongated tubular shaft (2) that is interiorly hollow provided on its free end with a defined geometric termination (20) that can be coupled in a region of the object to be handled (10) and a second elongated shaft (3) that passes through the inside of the first elongated shaft (2) and has on its end a defined geometric termination on its free end (30) that projects outwardly from the first elongated shaft (2), and can be coupled in a second region of the object to be handled in order to block it firmly. That is, the object to be handled and the geometric termination (30) rotate as one when they are coupled together.

In the present example, the tool (1) is intended to handle dental implants that have two perforated regions or orifices in their head, each of said perforated regions able to be coupled to a respective geometric termination that corresponds to each of the elongated shafts.
The second elongated shaft (3) is able to rotate on its own central longitudinal axis, in an arched trajectory of 30º for example, by using certain manual activation means explained below.

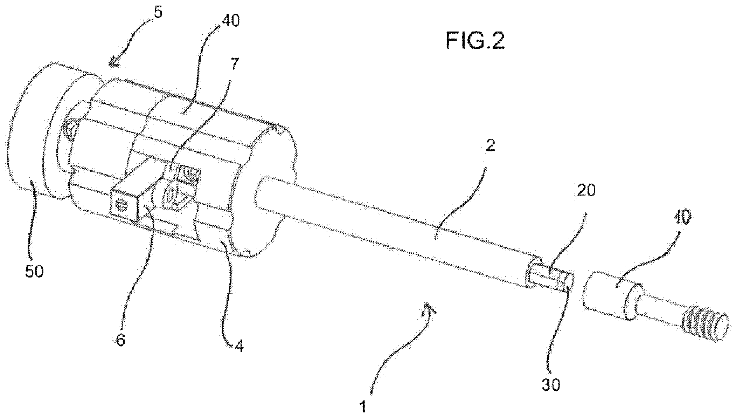

As shown in figures 1 and 2, the geometric termination (20) and the geometric termination (30), of the first and second shafts respectively, have a hexagonal contour (of the Allen type). In figure 1, the two ends (20, 30) are out of phase with each other, while in figure 2 these ends (20, 30) are aligned with each other.

In another alternative embodiment of the mechanical tool described above, it differs basically in the geometric shape of each of the geometric terminations (20, 30) of the elongated shafts (2) and (3) respectively, so as to be suitably adapted to the object to be handled.

Both the first (2) and the second (3) elongated shafts project outwardly from a protective casing (4) with the shape essentially of an elongated cylinder, made for example of rigid plastic, within which are the activation means, the protective casing (4) including a region (40) in the form of a handle with dimensions suited to facilitate manual handling of the mechanical tool (1).

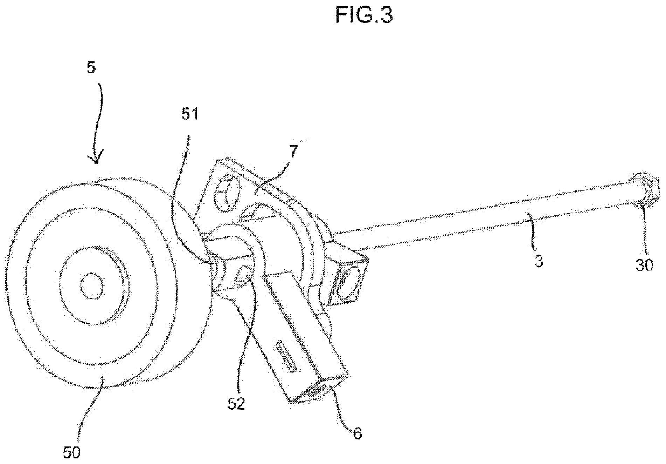

Referring now in particular to the means of activation, it has, as can be seen more clearly in figure 3, a push button (5) with a cylindrical body (50) and a stem or shaft (51) that projects from the posterior end of the protective casing (4) and which moves axially with respect to the protective casing (4) attached to the rotary shaft by a cam system, which is shown in greater detail in figure 3.

This cam system has a groove (52) with a curved trajectory placed in the stem (51) that projects from the cylindrical body (50), a converter element (6) having been coupled to the groove (52) by a pin (not shown) projecting outwardly, the converter element (6) being joined as a single piece to the rotatable elongated shaft, which makes it possible to transform the linear movement of the push-in element (5) into a rotary movement of the rotating shaft. In this case, the rotating shaft corresponds to the elongated shaft with the smaller diameter.

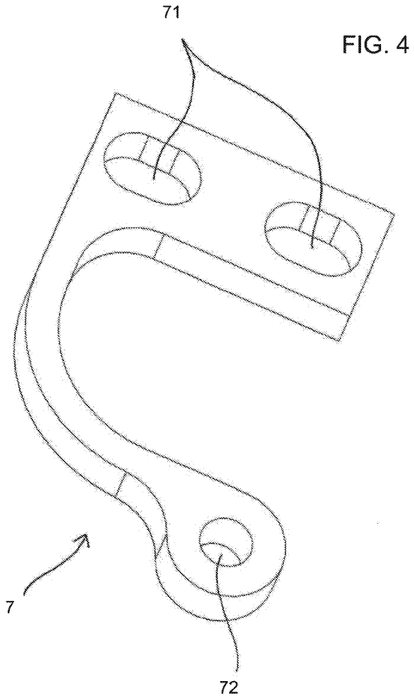

Moreover, elastic means attached to the above-described cam system are provided. These consist of a flexible element (7) formed by an essentially laminar body that is essentially U-shaped (see figure 4), which is joined to the converter element (6) and the rotary elongated shaft and is in a tensed state when the push button (5) has not been pushed in (moved toward the casing). In this case, the flexible element (7) is joined to the converter element (6) by a number of projections present on one side of the converter element (6) that fit under pressure into the corresponding pass-through holes located in the flexible element (7).

It should be noted that the flexible element (7) presents on one of it wings, a pair of slots (71) that pass through the laminar body and are suitable for the placement of screws that make it possible to adjust the position of the flexible element (7) inside the tool (1) and hence to regulate the degree of compression of the flexible element as a function of the position of the screw with respect to the path of the slot (71).

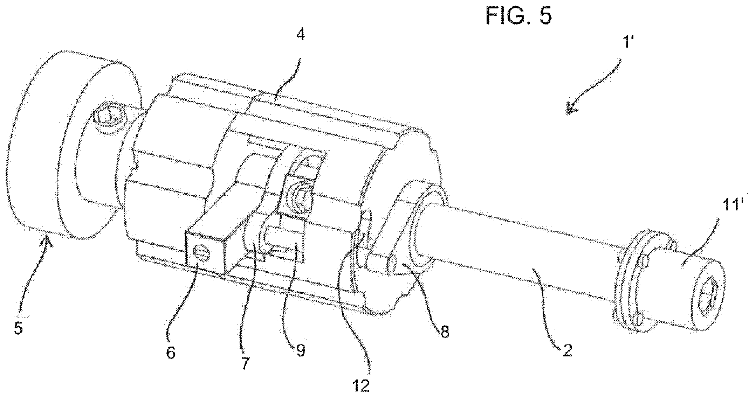

Figure 5 shows another preferred embodiment of the mechanical tool (1'), in which the elements that are in common with the previous embodiment have the same reference numbers. In this case, in contrast to the embodiment in figures 1 to 3, the rotary shaft is the one with the greater diameter and includes on its end an end (11') that has an interior orifice of a polygonal contour such as to accommodate the outside surfaces of the head of an implant (in distinction to the tool shown in figures 1 and 2 where the terminal part 11 is introduced into the inside of an orifice located on the head of the dental implant).

In order to cause the more exterior rotary shaft (2) to rotate, an attachment plate (8) is provided that is joined to the flexible element (7) by means of an elongated bar (9) adjusted to fit an orifice (72) of the same flexible element. This elongated bar (9) is guided by a groove (12) in the base of the protective casing (4), as shown in figure 5.

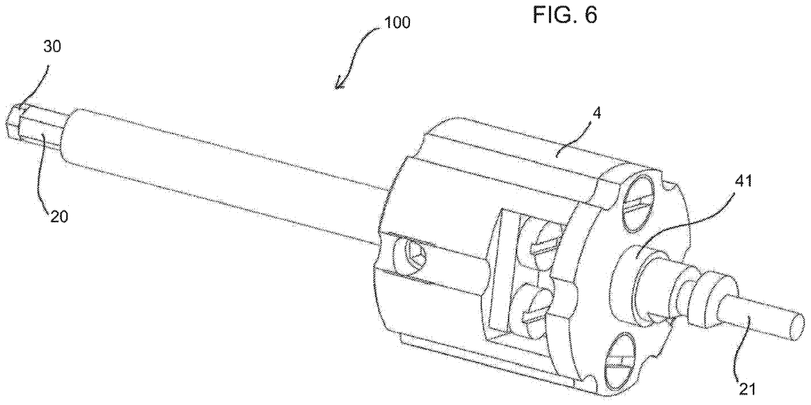

Figure 6 shows an alternative embodiment of the mechanical tool, indicated generally as with reference (1), in such a way that in this case the protective casing (4) has on its back end a coupling region (41) suitable for coupling to an electrically-operated tool (100), with the second elongated shaft (2) having an extension (21) in a direction opposite to the ends that project from the protective casing (4) so that they can be coupled to an electrically-supplied rotary actuator of known type (hence not described here in further detail), in distinction to the embodiment shown in figure 1, in which the activation is completely manual.

The details, shapes, dimensions and other accessory elements, as well as the materials used in the manufacture of the mechanical tool of the invention may be conveniently replaced by others that are technically equivalent and do not depart from the essential nature of the invention or from the scope as defined by the claims attached below.

## Claims

1. *A mechanical tool (1), for the manipulation of smaller-sized objects* such as dental implants, post-traumatic prostheses, screw, bolts, said mechanical tool *comprising a first elongated shaft (2) that is interiorly hollow having on its free end a defined geometric termination which can be coupled in a region of the object to be handled, and a second elongated shaft (3) that passes through the middle of the first elongated shaft and has on its free end a defined geometric termination (30) that projects outwardly from the first elongated shaft and can be coupled in a second region of the object to be handled, one of the two elongated shafts being able to rotate on its own central longitudinal axis in relation to the other elongated shaft, including an actuating means for carrying out said rotary action, **characterised by** the fact that the actuating means have a push button (5) comprising a* shaft (51) *that can be axially moved and it is coupled with the rotary shaft by means of a cam system.*

2. A mechanical tool (1) according to claim 1, **characterised by** the fact that the cam system has a groove (52) with a curved path placed in a shaft that projects from the push button (5), a converter element (6) joined as a unit to the rotatable elongated shaft having been coupled into the groove (52).

3. A mechanical tool (1) according to claim 1, **characterised by** the fact that it includes elastic means attached to the cam system.

4. A mechanical tool according to claim 3, **characterised by** the fact that the elastic means have a flexible element (7) comprised of an essentially laminar body that is joined to the converter element (6) and the rotating elongated shaft.

5. A mechanical tool (1) according to claim 4, **characterised by** the fact that the flexible element (7) includes at least one slot (71) that passes through the laminar body.

6. A mechanical tool (1) according to claim 1, **characterised by** the fact that the actuating means are housed inside a protective casing from which the first and second elongated shafts project.

7. A mechanical tool (1) according to claim 6, **characterised by** the fact that the protective casing has a region in the form of a handle.

8. A mechanical tool (1) according to claim 1, **characterised by** the fact that the geometric termination of the first elongated shaft has a polygonal exterior and/or interior contour.

9. A mechanical tool (1) according to claim 1, **characterised by** the fact that the geometric termination of the second elongated shaft has a polygonal exterior and/or interior contour.

10. A mechanical tool (1) according to claims 8 and 9, **characterised by** the fact that the geometric terminations of the first and second elongated shafts have the same polygonal exterior contour.

11. A mechanical tool (1) according to claim 6, **characterised by** the fact that the protective casing includes a coupling region suitable for coupling to an electrically-operated tool, the second elongated shaft having an extension in a direction opposite to the ends that project from the protective casing so that an electrically-supplied rotary activator may be coupled to it.

## Patentansprüche

1. Mechanisches Werkzeug (1), zur Manipulation von kleineren Objekten, wie Zahnimplantaten, posttraumatische Prothesen, Schrauben, Bolzen, wobei das mechanische Werkzeug eine erste längliche Welle (2), die innen hohl ist und an ihrem freien Ende einen definierten geometrischen Abschluss aufweist, der in einem Bereich des zu handhabenden Objekts gekoppelt werden kann, und eine zweite längliche Welle (3), die durch die Mitte der ersten länglichen Welle verläuft und an ihrem freien Ende einen definierten geometrischen Abschluss (30) aufweist, der von der ersten länglichen Welle nach außen ragt und in einem zweiten Bereich des zu handhabenden Objekts gekoppelt werden kann, umfasst, wobei eine der beiden länglichen Wellen in der Lage ist, sich um ihre eigene zentrale Längsachse in Bezug auf die andere längliche Welle zu drehen, mit einem Betätigungsmittel zur Durchführung der Drehbewegung,
**dadurch gekennzeichnet, dass** das Betätigungsmittel einen Druckknopf (5) aufweist, der eine axial bewegliche Welle (51) umfasst und mit der Drehwelle über ein Nockensystem gekoppelt ist.

2. Mechanisches Werkzeug (1) nach Anspruch 1, **dadurch gekennzeichnet, dass** das Nockensystem eine Nut (52) mit einem gekrümmten Weg aufweist, in einer Welle angeordnet, die vom Druckknopf (5) vorsteht, wobei ein Umwandlerelement (6), das als Einheit mit der drehbaren länglichen Welle verbunden ist, in die Nut (52) eingekoppelt wurde.

3. Mechanisches Werkzeug (1) nach Anspruch 1, **dadurch gekennzeichnet, dass** es federnde Mittel beinhaltet, die am Nockensystem befestigt sind.

4. Mechanisches Werkzeug nach Anspruch 3, **dadurch gekennzeichnet, dass** die federnden Mittel ein flexibles Element (7) aufweisen, das aus einem im Wesentlichen laminaren Körper besteht, der mit dem Umwandlerelement (6) und der drehenden länglichen Welle verbunden ist.

5. Mechanisches Werkzeug (1) nach Anspruch 4, **dadurch gekennzeichnet, dass** das flexible Element (7) mindestens einen Schlitz (71) aufweist, der durch den laminaren Körper verläuft.

6. Mechanisches Werkzeug (1) nach Anspruch 1, **dadurch gekennzeichnet, dass** die Betätigungsmittel in einem Schutzgehäuse untergebracht sind, aus dem die erste und zweite länglichen Wellen herausragen.

7. Mechanisches Werkzeug (1) nach Anspruch 6, **dadurch gekennzeichnet, dass** das Schutzgehäuse einen Bereich in Form eines Griffes aufweist.

8. Mechanisches Werkzeug (1) nach Anspruch 1, **dadurch gekennzeichnet, dass** der geometrische Abschluss der ersten länglichen Welle eine polygonale Außen- und/oder Innenkontur aufweist.

9. Mechanisches Werkzeug (1) nach Anspruch 1, **dadurch gekennzeichnet, dass** der geometrische Abschluss der zweiten länglichen Welle eine polygonale Außen- und/oder Innenkontur aufweist.

10. Mechanisches Werkzeug (1) nach den Ansprüchen 8 und 9, **dadurch gekennzeichnet, dass** die geometrischen Abschlüsse der ersten und zweiten länglichen Wellen die gleiche polygonale Außenkontur aufweisen.

11. Mechanisches Werkzeug (1) nach Anspruch 6, **dadurch gekennzeichnet, dass** das Schutzgehäuse einen Kopplungsbereich beinhaltet, der zum Koppeln mit einem elektrisch betriebenen Werkzeug geeignet ist, wobei die zweite längliche Welle eine Verlängerung in einer Richtung entgegengesetzt zu den Enden aufweist, die aus dem Schutzgehäuse herausragen, so dass ein elektrisch gespeister Drehantrieb daran gekoppelt werden kann.

## Revendications

1. Outil mécanique (1), pour la manipulation d'objets de petite taille tels que les implants dentaires, les prothèses post-traumatiques, les vis, les boulons, ledit outil mécanique
comprenant un premier arbre allongé (2) qui est creux à l'intérieur ayant à son extrémité libre une terminaison géométrique définie qui peut être couplée dans une région de l'objet à manipuler, et un deuxième arbre allongé (3) qui passe par le milieu du premier arbre allongé et qui a à son extrémité libre une terminaison géométrique définie (30) qui fait saillie vers l'extérieur du premier arbre allongé et qui peut être couplée dans une deuxième région de l'objet à manipuler, l'un des deux arbres allongés étant capable de tourner sur son propre axe central longitudinal par rapport à l'autre arbre allongé, comportant des moyens d'actionnement pour mettre en oeuvre ladite action rotative, **caractérisé par le fait que** les moyens d'actionnement ont un bouton poussoir (5) comprenant un arbre (51) qui peut être axialement déplacé et qui est couplé à l'arbre rotatif au moyen d'un système à came.

2. Outil mécanique (1) selon la revendication 1, **caractérisé par le fait que** le système à came a une rainure (52) avec une trajectoire incurvée placée dans un arbre qui fait saillie du bouton poussoir (5), un élément convertisseur (6) relié en tant qu'unité à l'arbre allongé rotatif ayant été couplé dans la rainure (52).

3. Outil mécanique (1) selon la revendication 1, **caractérisé par le fait qu'**il comprend des moyens élastiques fixés au système à came.

4. Outil mécanique selon la revendication 3, **caractérisé par le fait que** les moyens élastiques ont un élément flexible (7) constitué d'un corps essentiellement laminaire qui est relié à l'élément convertisseur (6) et à l'arbre allongé rotatif.

5. Outil mécanique (1) selon la revendication 4, **caractérisé par le fait que** l'élément flexible (7) comporte au moins une fente (71) qui passe par le corps laminaire.

6. Outil mécanique (1) selon la revendication 1, **caractérisé par le fait que** les moyens d'actionnement sont logés dans un boîtier de protection d'où les premier et deuxième arbres allongés font saillie.

7. Outil mécanique (1) selon la revendication 6, **caractérisé par le fait que** le boîtier de protection a une région sous la forme d'une poignée.

8. Outil mécanique (1) selon la revendication 1, **caractérisé par le fait que** la terminaison géométrique du premier arbre allongé a un contour polygonal extérieur et/ou intérieur.

9. Outil mécanique (1) selon la revendication 1, **caractérisé par le fait que** la terminaison géométrique du deuxième arbre allongé a un contour polygonal extérieur et/ou intérieur.

10. Outil mécanique (1) selon les revendications 8 et 9, **caractérisé par le fait que** les terminaisons géométriques des premier et deuxième arbres allongés ont le même contour polygonal extérieur.

11. Outil mécanique (1) selon la revendication 6, **caractérisé par le fait que** le boîtier de protection comporte une région de couplage apte pour le couplage à un outil à fonctionnement électrique, le deuxième arbre allongé ayant une extension dans un sens opposé aux extrémités qui font saillie du boîtier de protection de sorte qu'un activateur rotatif alimenté électriquement puisse être couplé à celui-ci.
